# EUROPEAN PATENT SPECIFICATION

(11) **EP 1 602 715 B1**
(45) Date of publication and mention of the grant of the patent: **11.06.2008**
(21) Application number: 05102682.1
(22) Date of filing: 05.04.2005
(51) Int. Cl.: C12M 1/00, B01L 3/00

(54) **Disposable bioreactor**
Einwegreaktor
Bioréacteur jetable

(30) Priority: 02.06.2004 US 576326
(43) Date of publication of application: 07.12.2005
(73) Proprietor: MILLIPORE CORPORATION, Billerica Massachusetts 01821 (US)
(72) Inventor: Proulx, Stephen, Boxboro, MA 01719 (US)
(74) Representative: Henkel, Feiler & Hänzel

(56) References cited:
- FR-A- 2 799 138
- GB-A- 2 202 549
- US-A- 5 565 015
- US-B1- 6 432 698

## Description

The present invention relates to the field of fermentation. More particularly it relates to a disposable fermenter or bioreactor having a built-in gas distributor and a device for inhibiting backflow or leakage through the gas distributor.

### BACKGROUND OF THE INVENTION

The biopharmaceutical industry has traditionally used stainless steel systems and piping in their manufacturing process as they are capable of being steam sterilized and reused.

The cost of such a system is often prohibitive. Moreover, such systems are static, often being welded together and not easily recanfigured.

The industry has begun to explore an alternative approach, namely to use plastic, single disposable bags and tubing to replace the traditional stainless steel. This allows one the flexibility to rearrange these systems at minimal cost. Additionally, the initial capital cost is several times less than that of stainless steel allowing one to manufacture biopharmaceuticals in smaller amounts, making available new therapeutic agents that prior to this advancement were not economically justified and allowing for the expansion of contract manufacturing of such products or when demand requires additional capacity quickly.

One aspect of the disposable biopharmaceutical plant has been the bioreactor, which needs a steady supply of gas and nutrients and removal of waste products and expelled gases. Additionally, a constant movement of the cells in the reactor helps to provide a constant mixing of the contents.

One system for a bioreactor has been to use a large table, equipped with motors or hydraulics onto which a bioreactor bag is placed. The motors/hydraulics rock the bag providing constant movement of the cells. Additionally, the bag has a gas and nutrient supply tube and waste gas and waste product tube which allow for the supply of nutrients and gases such as air for aerobic organisms and the removal of waste such as respired gases, carbon dioxide and the like. The tubes are arranged to work with the motion of the bag to allow for a uniform movement of the gases and fluids/solids. See U.S. Patent 6,191,913.

Such a system requires the use of capital-intensive equipment, with components that are susceptible to wear. Additionally, the size of the bag that can be used with the table is limited by the size of table and the lifting capability of its motors/hydraulics.

An alternative system uses a long flexible tube-like bag that has both ends attached to movable arms such that the bag after filling is suspended downwardly from the movable arms in the shape of a U. The arms are then alternately moved upward or downward relative to the other so as to cause a rocking motion and fluid movement within the bag. If desired the mid section may contain a restriction to cause a more intimate mixing action.

This system requires the use of a specially shaped bag and hydraulic or other lifting equipment to cause the movement of the liquid. Additionally, due to weight considerations, the bag size and volume is restricted by the lifting capacity of the equipment and the strength of the bag.

An improvement has been shown through the use of one or more bags that are capable of being selectively pressurized and deflated in conjunction with a disposable bio bag such as a fermenter, mixing bag, storage bag and the like. The pressure bag(s) may surround a selected outer portion of the bag or may be contained within an inner portion of such a bag. By selectively pressurizing and deflating the pressure bag(s), one is able to achieve fluid motion in the bag thereby ensuring cell suspension, mixing and/or gas and/or nutrient/ excrement transfer within the bag without damaging shear forces or foam generation.

Alternatively, one can select a static (non-moving) bag that contains a sparger or other device for introducing a gas into the bag. The gas causes the movement of the fluid in the bag as well to cause the mixing and transfer of gases, nutrients and waste products.

US 5,565,015 uses a flat, inflatable porous tube that is sealed into a plastic container. The tube inflates under gas pressure and allows gas to flow into the bag. When the gas is not applied the tube collapses and substantially closes off the pores of the flat tube to prevent leakage from the bag.

US 6,432,698, on which the preamble portion of claim 1 is based, also inserts and seals a tube to a gas diffuser within the bag. It appears that a constant positive gas pressure must be maintained in order to prevent any liquid within the bag from entering the diffuser and then the gas line and eventually the air pump as no valve or other means for preventing backflow is shown.

Both have the potential for leakage of the liquid in the container which can potentially contaminate the contents of the bag of the upstream components of the system such as the gas supply system. Additionally, both introduce a separate component for the gas distribution.

The present invention provides a device that overcomes these deficiencies.

### SUMMARY OF THE INVENTION

The present invention is a bioreactor or fermenter as defined in claim 1.The hydrophobic filter acts to purify the gas entering the container so as to prevent the introduction of contaminants such as microbes into the container. It also acts as a valve, preventing liquid in the bag from passing through its hydrophobic structure thus preventing any back flow when positive pressure from the gas supply is not being imposed upon the bag. The distribution device is preferably formed such as by bonding the two facing wall surfaces of the container together at discrete and spaced apart locations to form a series of gas ports into the remainder of the container.

In one embodiment, the device is formed of flexible or semi-rigid plastic with its one or more wall edges sealed together to form a closed container. The one or more inlets and outlets can either be formed in the plastic as made or they can be added as needed with the appropriate sealing.

In another embodiment, the bottom wall of the container has an upward taper, either to one side or both sides or a rounded bottom wall to eliminate any deadspace in corners.

In a further embodiment the container is formed of semi-rigid plastic in a two piece clam shell design that is sealed to each other by their adjacent wall edges.

### IN THE DRAWINGS

Figure 1 shows a cross-sectional view of a first embodiment of the present invention.
Figure 2A shows a cross-sectional view of a second embodiment of the present invention.
Figure 2B shows a cross-sectional view of another embodiment of the present invention.
Figure 3 shows a cross-sectional view of another embodiment of the present invention.
Figure 4 shows a cross-sectional view of another embodiment of the present invention.
Figure 5 shows a cross-sectional view of another embodiment of the present invention.
Figure 6 shows a cross-sectional view of another embodiment of the present invention.

### DETAILED DESCRIPTION

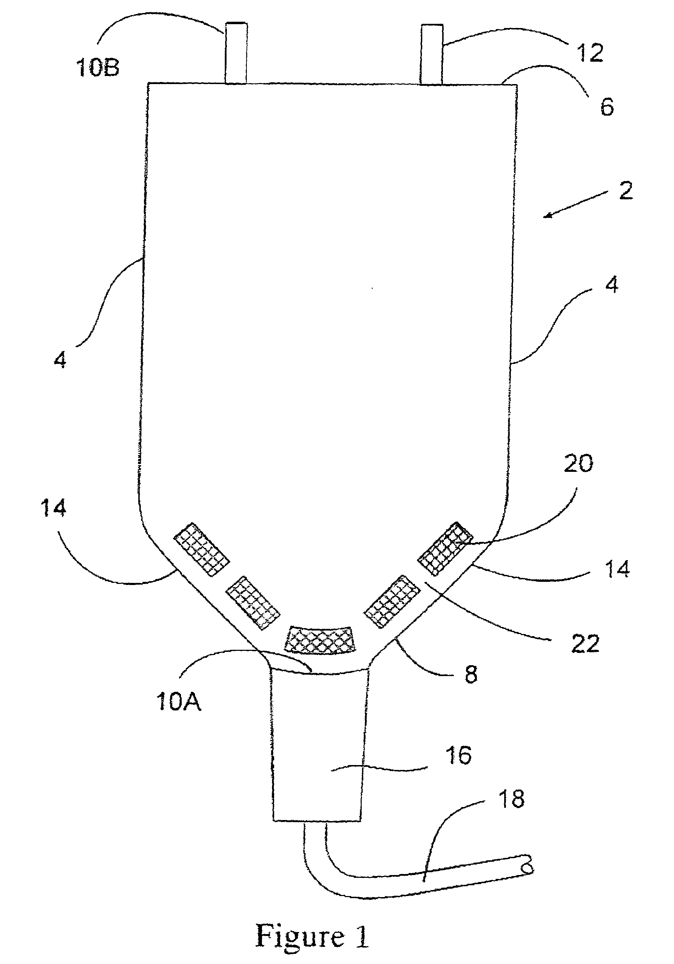

The present invention relates to a disposable fermenter or bioreactor. Figure 1 shows a first embodiment of the present invention. As shown the device is comprised of a bag 2 having two substantially vertical sidewalls 4, a top wall 6 and a bottom wall 8. One or more inlets 10A-B and one or more outlets 12 are formed one or more walls of the in the bag 2. One inlet for gas 10A is located at the lowermost portion of the bottom wall 8 of the container 2. Attached to the inlet 10A and upstream of the inlet 10A is a hydrophobic filter 16. Connected to the other end of the filter 16 is a gas line 18 for supplying air and/or other gases to the container 2. A series of one or more gas distribution devices 20 are formed in the container adjacent the bottom wall 8. The one or more devices are designed to allow gas to enter the container adjacent the inlet 10A. The devices form one or more gas ports 22 which distribute the gas through out the container before it enters the remainder of the container. Preferably as shown, there are a plurality of these devices 20 that form a series of two or more gas ports 22 so as to maximize this distribution effect.

The one or more inlets 10 and outlets 12 may be formed as part of the container itself or they may be attached to the bag and liquid tightly sealed to the walls so as to prevent any leakage. The use of plastic tubes, hose barbs and luer fittings that can be sealed to the container wall(s) are the most common devices that can be used to form the one or more inlets and outlets, Others will also be readily apparent and are meant to be incorporated herein. They may be sealed to the wall(s) by heat sealing or vibration welding, adhesives, solvents, overmolding of plastic seals and the like, as are commonly used in the container industry.

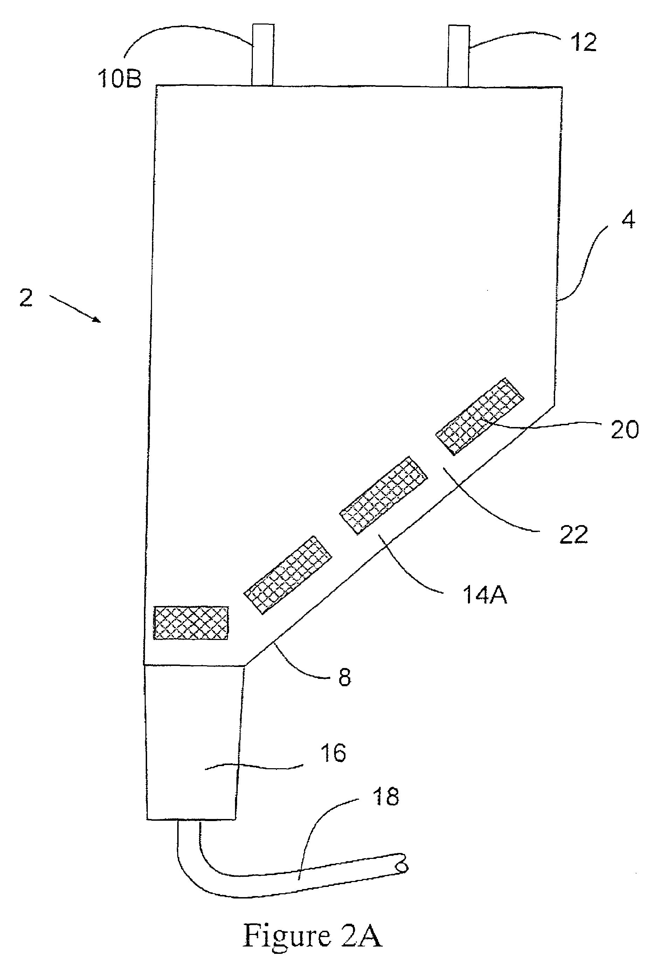

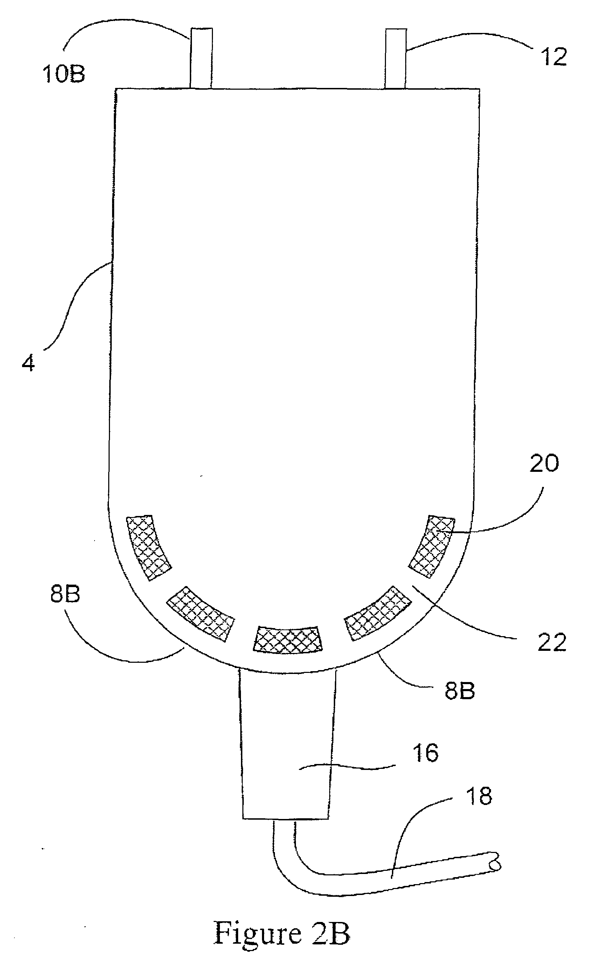

As shown in Figure 1, the bottom wall 8 has an upward taper 14 to each of the sidewalls 4. This is used to avoid the formation of dead zones for circulation within the container. While this is a preferred design, a flat bottom wall may also be used, especially if the mixing is deemed to be adequate or not necessary to its use. Other alternative embodiments include a bottom wall 8 having a single upward linear taper 14A toward one of the side walls 4 as shown in Figure 2A or it may have a rounded bottom wall 8B as shown in Figure 2B. Other designs may also be used.

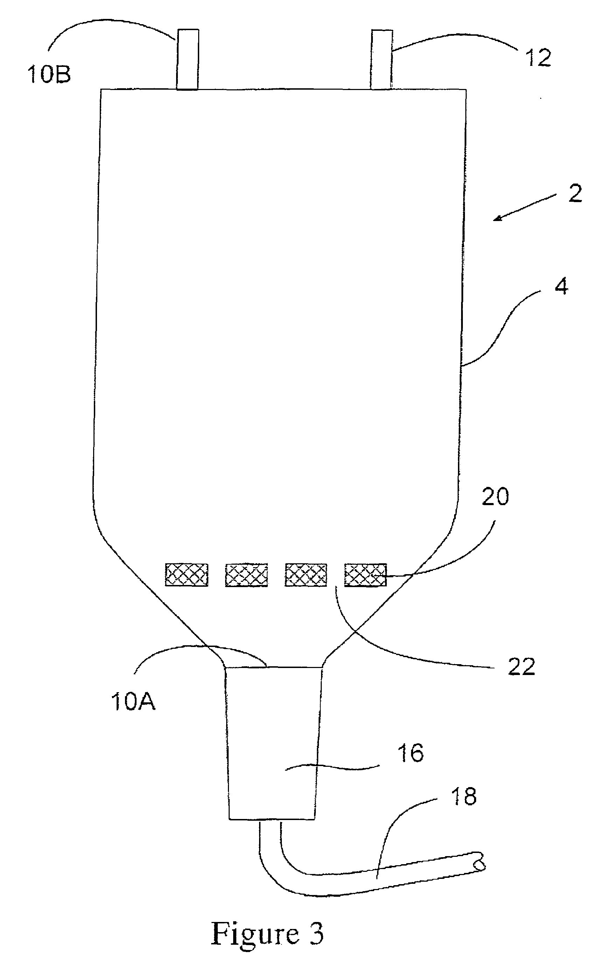

The one or more gas distribution devices 20 are formed from the walls of the container itself. This can be accomplished for example by selectively sealing adjacent, opposite wall portions together such as by the use of heat sealing, vibration welding, adhesives and the like so they are permanently attached to each other and gas must flow around them in order to rise up through the container. Preferably, the arrangement of the devices conforms to the shape of the bottom wall 8. Alternatively as shown in Figure 3 they may be arranged in a horizontal row regardless of the bottom wall 8 configuration.

Any hydrophobic membrane containing filter may be used in the present invention. The membrane may be inherently hydrophobic such as polyvinylidene fluoride (PVDF), polyethersulfone (PES) or PTFE resin or it may be treated, such as by a crosslinked coating or graft polymerization of a surface treatment, either to render the polymer hydrophobic or to provide the desired level of hydrophobicity. Such filters are well-known in the art and include for example DURAPORE® filter cartridge filters or AERVENT® hydrophobic cartridge filters available from Millipore Corporation of Billerica, Massachusetts. The filter provides duel functionality in the present invention. It acts as a filter for any gas entering the fermenter to keep dust, particles or bacteria (when one selects a filter having a nominal pore size of less than 0.22 micron) from entering and potentially contaminating the fermenter. More importantly, as it is hydrophobic, it will prevent liquid from leaving the fermenter through the gas inlet in the event that either the gas pressure is off or is lower than the static or head pressure applied by the liquid in the fermenter. In most applications, selecting a suitably sized filter with the desired level of hydrophobicity also means that one need not use a valve between the fermenter and the gas supply and can use the inherent resistance of the filter's membrane to act as a valve instead. For example, a DURAPORE® filter cartridge is capable of withstanding about 206.84 KPa (30 psi) pressure differential of water meaning that it is capable of supporting a large column of water or similar liquids. The key is to select a filter and use it with a volume of liquid such that the head pressure of the liquid in the fermenter doesn't meet or exceed the intrusion pressure of the membrane within the filter cartridge.

The container can be formed of two flat sheets of material which are heat bonded, vibration welded, adhered or solvated along their adjacent outer edges. Alternatively, it can be formed of a plastic tube so that only the top 6 and bottom wall 8 need to seal to each other. In another embodiment, a single sheet may be used and folded in half on itself so that the sidewalls 4 and top wall 6 need to be sealed together.

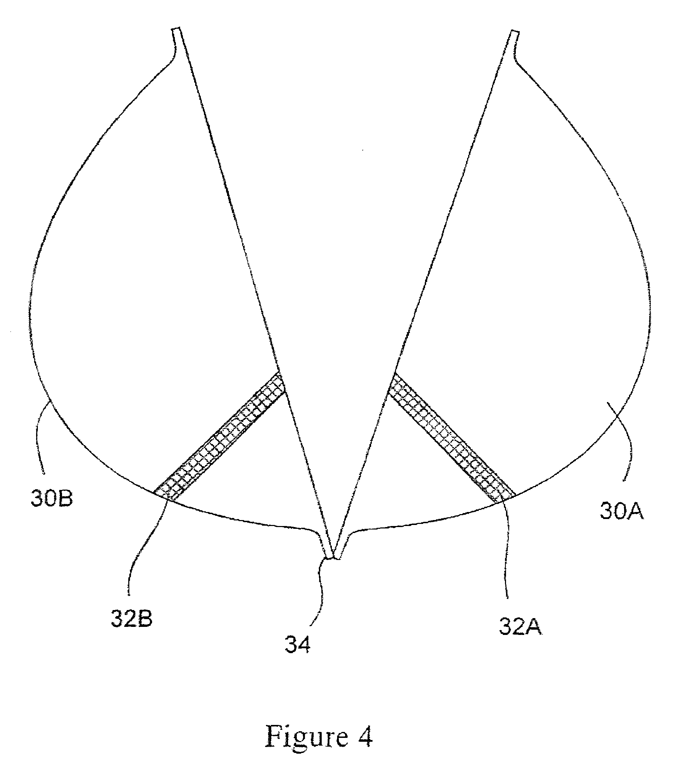

In the embodiment of Figure 4, the container is formed of two molded halves, 30A and B that are then sealed to other along their adjacent outer edges. The gas distributing devices 32A and 32B are also molded into the respective halves 30A and 30B. When the two halves 30A and B are sealed together the two distributor halves 32A and B are also preferably in contact with each other. If desired they may be sealed to other as well by heat, solvents, adhesives or vibration welding. Alternatively, they may be molded with features that interlock to each other such as male/female portions (e.g. key and keyway) on the respective halves to hold them together.

Preferably in the design of the embodiment of Figure 4, the device is a single molded piece connected by a hinged portion 34 as shown. This is commonly referred to as a clamshell housing. Alternatively, they can be two separate pieces if desired.

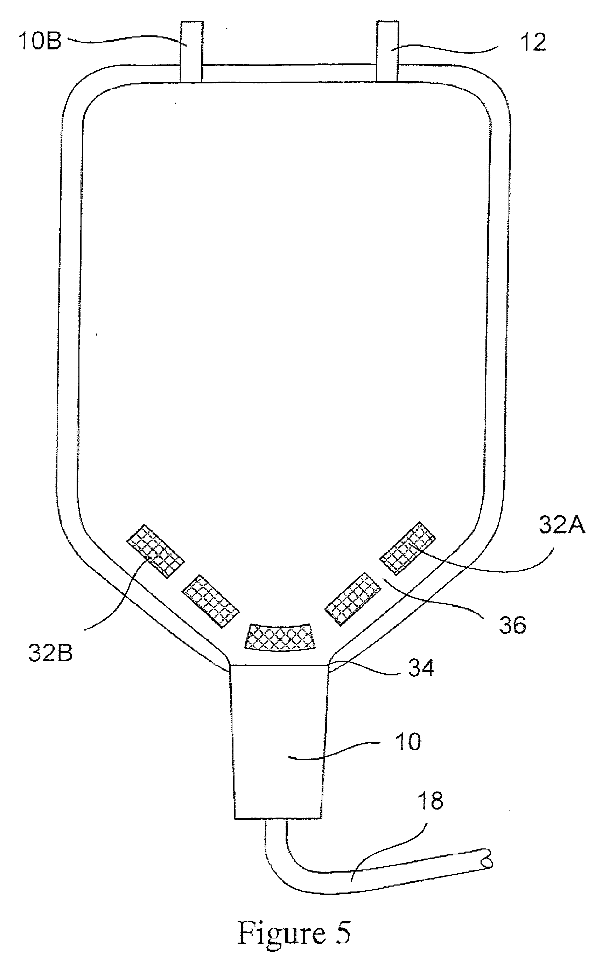

Figure 5 shows the design of Figure 4 in a face on planar view after assembly. As can be seen it is essentially identical in design to that of Figures 1-3 and operates in the same manner. Also shown are the gas ports 36 between the gas distribution devices 32 A/B.

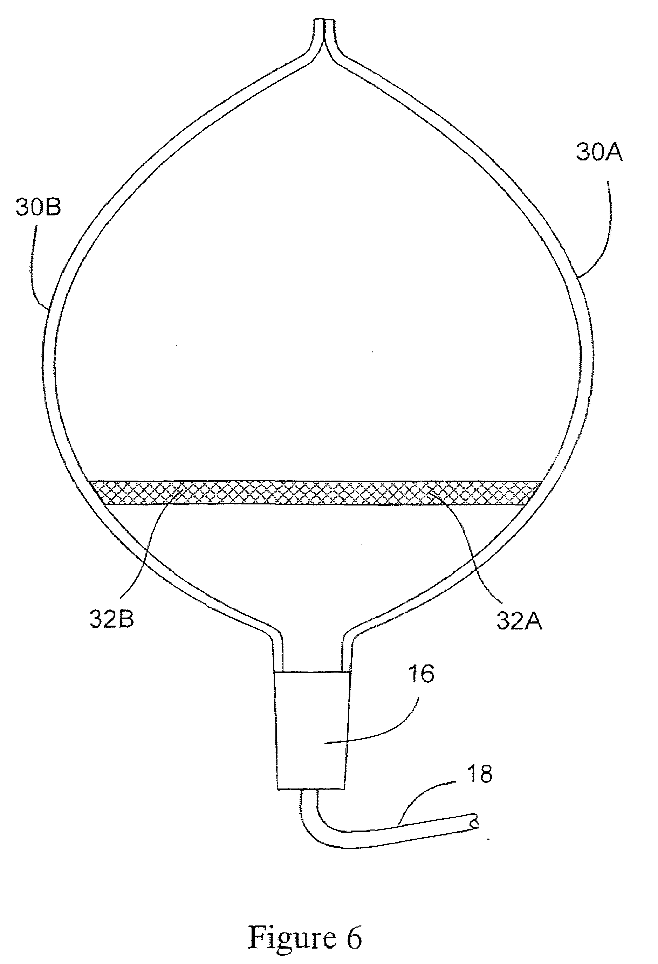

Figure 6 shows the design of Figure 4 in a cross-sectional view through the centerline after assembly. As can be seen it is essentially identical in design to that of Figures 1-3 and operates in the same manner. While not shown due the cross sectional slice chosen, the gas ports 36 exist on either side of the distribution device 32A/B.

The materials used in the present invention to make the container can be those typically used in the biopharmaceutical industry for disposable bioreactors, fermenters, storage bags and the like. Such materials and/or bags are available from a variety of suppliers such as Stedim SA of France and Hyclone of Logan, Utah. These bags range in size from a few liters to 2000 liters or more. They typically are made from multiple layered (extruded or laminated) plastic film such as polyethylene, polypropylene, EVA copolymers, EVOH, PET, PETG, specialty or proprietary polymers such as the HyQ CX5-14 film available from Hyclone which is a coextruded multilayer film with an outer layer of elastomer with an EVOH barrier layer and an ultra-low density polyethylene product contact layer, blends of polymers and the like. The polymer(s) selected are chosen for the desired combination of cleanliness, strength and visibility.

When used in the clam shell format of Figure 4-6, one can use polymers that are capable of being formed by vacuum or injected molded such as polyethylene, polypropylene, polycarbonates, polystyrenes, various acrylics and methacrylics and copolymers thereof, and the like.

The present invention provides one with a simple system for the growth of microbes in a fermenter or bioreactor as a disposable system with good gas and fluid distribution. It allows one to achieve the adequate movement and /or mixing of components as desired without the need for capital and maintenance intensive equipment such as rocking tables or hydraulic hoists or cranes. While contemplated for use in the blopharmaceutical industry, it is clear that the device of the present invention has applications in other fields such as beer brewing, wine making and the like in disposable bags.

## Claims

1. A fermenter comprising
a plastic container having one or more sealed walls,
one or more inlets (10A,B) and one or more outlets (12) that are formed in the one or more walls of the container,
at least one (10A) of the one or more inlets (10A, B) is formed in a bottom wall (8) of the container and is adapted to be connected to a source of gas,
one or more gas distribution devices (20;32A,B) formed within the body of the container adjacent said at least one gas inlet (10A), and
a filter (16) mounted between said at least one gas inlet (10A) and the source of the gas,
**characterized in that**
said filter (16) is hydrophobic.

2. The fermenter of claim 1 wherein the one or more gas distribution devices (20;32A,B) are formed by bonding two facing wall surfaces of the container together at one or more locations to form one or more gas ports (22;36) into the remainder of the container.

3. The fermenter of claim 2 wherein the one or more gas distribution devices (20;32A,B) are formed by bonding the two facing wall surfaces of the container together at two or more discrete locations spaced apart from each other so as to form a series of two or more gas ports (22;36) into the remainder of the container.

4. The fermenter of any one of claims 1 to 3 wherein the bottom wall (8) of the container is upwardly tapered toward one or more of the sidewalls of the container.

5. The fermenter of any one of claims 1 to 4 wherein the bottom wall (8) of the container is upwardly tapered in the form of a V.

6. The fermenter of any one of claims 1 to 4 wherein the bottom wall (8) of the container is tapered in the form of a rounded shape toward the sidewalls.

7. The fermenter of any one of claims 1 to 6 wherein the plastic container is a flexible plastic bag.

8. The fermenter of any one of claims 1 to 6 wherein the plastic container is a molded plastic container.

9. The fermenter of any one of claims 1 to 6 wherein the plastic container is a molded plastic container comprised of two halves that are sealed to each other along their adjacent outer edges.

10. The fermenter of any one of claims 1 to 6 wherein the plastic container is a molded plastic container comprised of two halves connected together by a hinge portion and the two halves are sealed to each other along their adjacent outer edges.

11. The fermenter of any one of claims 1 to 10 wherein the hydrophobic filter (16) has an average pore size of about 0.22 micron and a bubble point of at least 137.89 kPa (20 psi), preferably of at least 172.37 kPa (25 psi), more preferably of at least 206.84 kPa (30 psi).

12. The fermenter of any one of claims 1 to 11 wherein said hydrophobic filter (16) has a membrane that is selected so as to be sized and have a level of hydrophobicity such that; in operation, a head pressure of a rated use volume of liquid in the container is smaller than an intrusion pressure of the membrane of the filter (16).

## Patentansprüche

1. Fermenter mit
einem Kunststoffbehälter mit einer oder mehreren abgedichteten Wänden,
einem oder mehreren Einlass/Einlässen (10A,B) und einem oder mehreren Auslass/Auslässen (12), die in der einen oder den mehreren Wänden des Behälters ausgebildet ist/sind,
wobei mindestens einer (10A) des einen oder der mehreren Einlässe (10A, B) in einer Bodenwand (8) des Behälters ausgebildet ist und mit einer Gasquelle verbunden werden kann,
einer oder mehreren Gasverteilungsvorrichtungen (20;32A,B), die in dem Körper des Behälters angrenzend an den mindestens einen Gaseinlass (10A) ausgebildet ist/sind, und
einen zwischen dem mindestens einen Gaseinlass (10A) und der Gasquelle angebrachten Filter (16),
**dadurch gekennzeichnet, dass**
der Filter (16) hydrophob ist.

2. Fermenter nach Anspruch 1, wobei die eine oder die mehreren Gasverteilungsvorrichtung(en) (20;32A,B) durch miteinander Verbinden bzw. Verkleben von zwei gegenüberliegenden Wandflächen des Behälters an einer oder mehreren Stellen gebildet ist/sind, um einen oder mehrere Gasöffnung(en) (22;36) in den Rest des Behälters auszubilden.

3. Fermenter nach Anspruch 2, wobei die eine oder mehreren Gasverteilungsvorrichtung(en) (20;32A,B) durch miteinander Verbinden bzw. Verkleben der zwei gegenüberliegenden Wandflächen des Behälters an zwei oder mehreren diskreten, voneinander beabstandeten Stellen gebildet ist/sind, so dass eine Reihe von zwei oder mehreren Gasöffnungen (22;36) in den Rest des Behälters ausgebildet sind.

4. Fermenter nach einem der Ansprüche 1 bis 3, wobei die Bodenwand (8) des Behälters nach oben hin zu einer oder mehreren der Seitenwände des Behälters schräg verläuft.

5. Fermenter nach einem der Ansprüche 1 bis 4, wobei die Bodenwand (8) des Behälters in der Form eines V nach oben schräg verläuft.

6. Fermenter nach einem der Ansprüche 1 bis 4, wobei die Bodenwand (8) des Behälters in einer abgerundeten Form zu den Seitenwänden hin abgeschrägt ist.

7. Fermenter nach einem der Ansprüche 1 bis 6, wobei der Kunststoffbehälter ein flexibler Kunststoffbeutel ist.

8. Fermenter nach einem der Ansprüche 1 bis 6, wobei der Kunststoffbehälter ein geformter ("molded") bzw. gespritzter Kunststoffbehälter ist.

9. Fermenter nach einem der Ansprüche 1 bis 6, wobei der Kunststoffbehälter ein geformter ("molded") bzw. gespritzter Kunststoffbehälter ist, der aus zwei Hälften gebildet ist, die miteinander entlang ihren aneinandergrenzenden Außenrändern versiegelt sind.

10. Fermenter nach einem der Ansprüche 1 bis 6, wobei der Kunststoffbehälter ein geformter("molded") bzw. gespritzter Kunststoffbehälter ist, der zwei Hälften aufweist, die durch einen Scharnierabschnitt miteinander verbunden sind, wobei die beiden Hälften entlang ihren aneinandergrenzenden Außenrändern miteinander versiegelt sind.

11. Fermenter nach einem der Ansprüche 1 bis 10, wobei der hydrophobe Filter (16) eine durchschnittliche Porengröße von etwa 0,22 Mikron und einen Blasenpunkt von mindestens 137,89 kPA (20 psi) aufweist, vorzugsweise von mindestens 172,37 kPa (25psi) und am bevorzugtesten von mindestens 206,84 kPa (30psi).

12. Fermenter nach einem der Ansprüche 1 bis 11, wobei der hydrophobe Filter (16) eine Membran hat, die derart gewählt ist, dass sie so bemessen ist und ein solches Niveau an wasserabweisender Eigenschaft aufweist, dass im Betrieb ein Kopfdruck eines Nenn-Flüssigkeitsnutzvolumens in dem Behälter geringer ist als ein Intrusionsdruck der Membran des Filters (16).

## Revendications

1. Fermenteur comprenant
un récipient en matière plastique comportant une ou plusieurs parois scellées,
une ou plusieurs entrées (10A, B) et une ou plusieurs sorties (12) qui sont formées dans les une ou plusieurs parois du récipient,
au moins une (10A) des une ou plusieurs entrées (10A,
B) est formée dans une paroi inférieure (8) du récipient et est conçue pour être reliée à une source de gaz,
un ou plusieurs dispositifs de distribution de gaz (20 ; 32A, B) formés à l'intérieur du corps du récipient adjacent à ladite au moins une entrée de gaz (10A), et
un filtre (16) monté entre ladite au moins une entrée de gaz (10A) et la source du gaz,
**caractérisé en ce que**
ledit filtre (16) est hydrophobe.

2. Fermenteur selon la revendication 1, dans lequel les un ou plusieurs dispositifs de distribution de gaz (20 ; 32A, B) sont formés en reliant deux surfaces de parois opposées du récipient ensemble à un ou plusieurs emplacements afin de former un ou plusieurs orifices de gaz (22 ; 36) dans le reste du récipient.

3. Fermenteur selon la revendication 2, dans lequel les un ou plusieurs dispositifs de distribution de gaz (20 ; 32A, B) sont formés en reliant les deux surfaces de parois opposées du récipient ensemble à deux emplacements distincts ou plus espacés l'un de l'autre de manière à former une série de deux orifices de gaz ou plus (22 ; 36) dans le reste du récipient.

4. Fermenteur selon l'une quelconque des revendications 1 à 3, dans lequel la paroi inférieure (8) du récipient est inclinée vers le haut vers une ou plusieurs des parois latérales du récipient.

5. Fermenteur selon l'une quelconque des revendications 1 à 4, dans lequel la paroi inférieure (8) du récipient est effilée vers le haut en la forme d'un V.

6. Fermenteur selon l'une quelconque des revendications 1 à 4, dans lequel la paroi inférieure (8) du récipient est effilée pour présenter une forme arrondie vers les parois latérales.

7. Fermenteur selon l'une quelconque des revendications 1 à 6, dans lequel le récipient en matière plastique est un sac en matière plastique souple.

8. Fermenteur selon l'une quelconque des revendications 1 à 6, dans lequel le récipient en matière plastique est un récipient en matière plastique moulé.

9. Fermenteur selon l'une quelconque des revendications 1 à 6, dans lequel le récipient en matière plastique est un récipient en matière plastique moulé constitué de deux moitiés qui sont scellées l'une à l'autre le long de leurs bords extérieurs adjacents.

10. Fermenteur selon l'une quelconque des revendications 1 à 6, dans lequel le récipient en matière plastique est un récipient en matière plastique moulé constitué de deux moitiés reliées ensemble par une partie d'articulation et les deux moitiés sont scellées l'une à l'autre le long de leurs bords extérieurs adjacents.

11. Fermenteur selon l'une quelconque des revendications 1 à 10, dans lequel le filtre hydrophobe (16) présente une taille de pore moyenne d'environ 0,22 micromètre et un point de bulle d'au moins 137,89 kPa (20 livres par pouce carré), de préférence d'au moins 172,37 kPa (25 livres par pouce carré), de façon davantage préférée d'au moins 206,84 kPa (30 livres par pouce carré).

12. Fermenteur selon l'une quelconque des revendications 1 à 11, dans lequel ledit filtre hydrophobe (16) comporte une membrane qui est sélectionnée de manière à être dimensionnée et à avoir un niveau de caractère hydrophobe tel que, en fonctionnement, une pression de refoulement d'un volume d'utilisation nominal de liquide dans le récipient est inférieure à une pression d'intrusion de la membrane du filtre (16).
